# EUROPEAN PATENT SPECIFICATION

(11) **EP 2 116 173 B1**
(45) Date of publication and mention of the grant of the patent: **16.11.2016**
(21) Application number: 09006091.4
(22) Date of filing: 04.05.2009
(51) Int. Cl.: A61B 1/045, A61B 5/00, A61B 5/07, A61B 1/04, A61B 1/05, A61B 1/00

(54) **Capsule medical apparatus**
Kapselförmige medizinische Vorrichtung
Appareil médical de type capsule

(30) Priority: 09.05.2008 JP 2008123534
(43) Date of publication of application: 11.11.2009
(73) Proprietor: Olympus Corporation, Tokyo 192-8507 (JP)
(72) Inventor: Takenaka, Tomoya, Oita-shi Oita 870-0126 (JP); Homan, Masatoshi, Hachioji-shi, Tokyo 192-8507 (JP)
(74) Representative: Schicker, Silvia

(56) References cited:
- WO-A-2004/059568
- WO-A-2007/060656
- WO-A1-2008/032713

## Description

### TECHNICAL FIELD

The present invention relates to a capsule medical apparatus introduced into an organ of a subject such as a patient, for obtaining in-vivo information of the subject.

### BACKGROUND ART

A swallowable capsule medical apparatus equipped with an imaging function and a wireless communication function has been used in the medical field. In general, the capsule medical apparatus is swallowed from the mouth of a subject for observation (examination) of an inside of the subject, thereafter sequentially takes an image (hereinafter, also referred to as an in-vivo image) inside the organ of the subject while traveling in the organ of the subject by peristaltic action or the like, and sequentially wirelessly transmits an image signal of the obtained in-vivo image to outside of the subject. The capsule medical apparatus sequentially repeats taking of the in-vivo image of such a subject and wireless transmission of the image signal during a time period until naturally excreted to the outside of the subject.

The image signal wirelessly transmitted by the capsule medical apparatus in the subject is received by a receiving device outside the subject. The receiving device has a receiving antenna arranged on a body surface of the subject and receives the image signal from the capsule medical apparatus through the receiving antenna. Further, a predetermined recording medium is inserted to the receiving device in advance, and the receiving device sequentially records the in-vivo image of the subject transmitted from the capsule medical apparatus, in the recording medium.

The recording medium in the receiving device is detached from the receiving device after the capsule medical apparatus is naturally excreted to the outside of the subject, and inserted to a predetermined image display device. The image display device takes in an image data group in the recording medium, i.e., an in-vivo image group of the subject taken by the capsule medical apparatus, and displays the obtained in-vivo image group on a display. A user such as a doctor and a nurse observes the in-vivo image group displayed by the image display device to diagnose the subject.

As one type of the capsule medical apparatus, a floatable in-vivo imaging device, which travels through a body cavity, for example, is described in Published Japanese Translation No. 2007-516765 (Kohyo) of the PCT International Publication. The in-vivo imaging device is swallowed by a patient and thereafter floats on liquid in a stomach of the patient. The in-vivo imaging device in the stomach takes an image in the stomach while floating on the liquid and transmits the obtained image data to an external receiving unit.

The image signal wirelessly transmitted by the capsule medical apparatus in the subject is generally received by the external receiving device through a living tissue of the subject (in other words, a human body). Therefore, it is possible that a part of an electric wave including the imaging signal from the capsule medical apparatus is reflected by an inner wall portion of the human body. Due to this, there has been a problem that it is difficult to improve an electric wave propagation property between the capsule medical apparatus in the subject and the receiving device outside the subject.

WO 2007/060656 A discloses a device for in-vivo imaging of a body. The device includes two transparent elongated optical domes, behind which illumination sources are situated, two imagers and a transmitter. An antenna for transmitting and receiving video signals from the imagers is provided. The whole antenna is always above or below the liquid surface. The device communicates with an external receiving and display system to provide display of data, control or other functions. The center of gravity of the device is adjusted to ensure that one side of the device will remain afloat while the other side will be submerged when moving through the liquid. The center of gravity may be set in a section or in proximity to a section of the device that is desired to be submerged during the device movement. Accordingly, the center of gravity may be determined by positioning certain components of the device in desired areas or designing the device in a way that ensures that the specific weight of the device is compatible to the shape of the outer envelope. Thus, both the head and tail of the device, such as the domes, are disposed at an angle perpendicular to the fluid.

WO 2004/059568 A1 discloses an in-vivo imaging device including at least one sensor such as an image sensor. The image sensor, illumination sources and a transmitter are mounted on a support so as to minimize the amount of space required by such components. An antenna may be placed within or mounted on a surface of an optical isolation element. The antenna can be coiled within or embedded within the isolation element.

### DISCLOSURE OF INVENTION

A capsule medical apparatus according to one aspect of the present invention floats on a liquid surface of liquid introduced into a subject, and includes at least one in-vivo information obtaining unit that obtains in-vivo information of the subject, a transmitting unit that wirelessly transmits an electric wave including the in-vivo information obtained by the at least one in-vivo information obtaining unit to outside through a transmitting antenna, and a capsule-type casing having the at least one in-vivo information obtaining unit, the transmitting antenna, and the transmitting unit fixedly arranged therein. The transmitting antenna is arranged on a liquid surface position of the liquid when a part of the capsule-type casing floats on the liquid surface of the liquid.

The above and other objects, features, advantages and technical and industrial significance of this invention will be better understood by reading the following detailed description of presently preferred embodiments of the invention, when considered in connection with the accompanying drawings.

### BRIEF DESCRIPTION OF DRAWINGS

FIG. 1 is a schematic diagram of one configuration example of an intra-subject information acquiring system having a capsule medical apparatus according to an embodiment of the present invention;
FIG. 2 is a cross-sectional schematic diagram of one configuration example of the capsule medical apparatus according to the embodiment of the present invention;
FIG. 3 is a schematic diagram for illustrating a transmitting antenna of the capsule medical apparatus according to the embodiment of the present invention;
FIG. 4 is a schematic diagram for illustrating a state in which the capsule medical apparatus in a floating state transmits an electric wave to an external receiving antenna through the transmitting antenna; and
FIG. 5 is a schematic diagram for illustrating another mode of a transmitting antenna arrangement.

### BEST MODE(S) FOR CARRYING OUT THE INVENTION

Hereinafter, a preferred embodiment of a capsule medical apparatus according to the present invention is described in detail with reference to the drawings. Although a capsule medical apparatus for obtaining an in-vivo image, which is one example of in-vivo information of a subject, is hereinafter illustrated as one example of the capsule medical apparatus according to the present invention, the present invention is not limited to the embodiment.

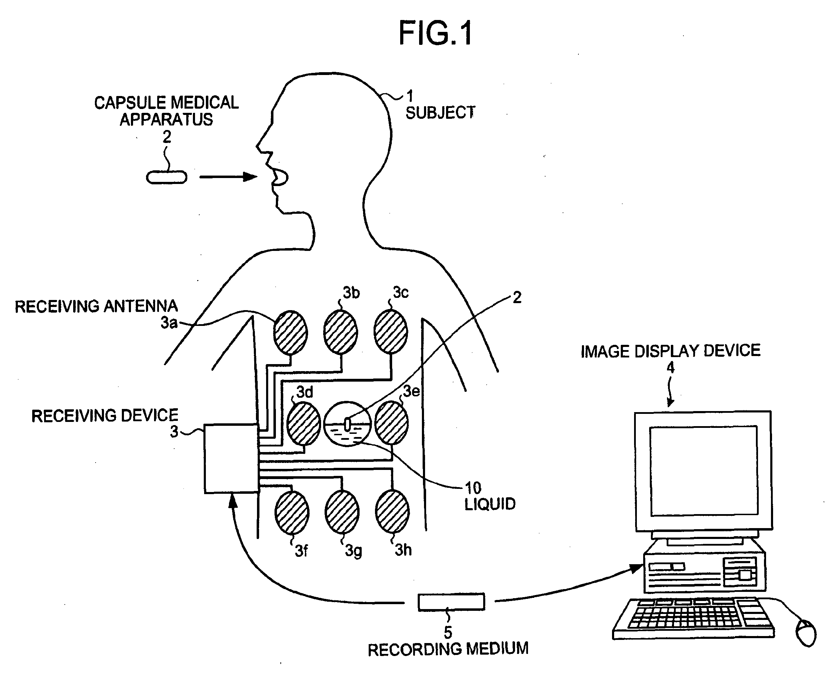

FIG. 1 is a schematic diagram of one configuration example of an intra-subject information acquiring system having the capsule medical apparatus according to the embodiment of the present invention. The intra-subject information acquiring system according to the embodiment is for obtaining an in-vivo image, which is one example of the in-vivo information of a subject 1. Specifically, as illustrated in FIG. 1, the intra-subject information acquiring system includes a capsule medical apparatus 2 that takes the in-vivo image of the subject 1, a receiving device 3 that receives an image signal of the subject 1 wirelessly transmitted by the capsule medical apparatus 2, an image display device 4 that displays the in-vivo image of the subject 1, and a recording medium 5 that passes data between the receiving device 3 and the image display device 4.

The capsule medical apparatus 2 is a capsule-type medical apparatus equipped with an imaging function and a wireless communication function, for taking the in-vivo image of the subject 1. Specifically, the capsule medical apparatus 2 is introduced into the subject 1 through oral intake or the like, then travels in a digestive tract of the subject 1 by peristaltic action or the like, and finally naturally excreted to outside of the subject 1. The capsule medical apparatus 2 sequentially takes the in-vivo image of the subject 1 at a predetermined time interval (for example, at 0.5-second intervals) and sequentially wirelessly transmits the image signal including the obtained in-vivo image to the external receiving device 3 until naturally excreted to the outside of the subject 1.

Also, the capsule medical apparatus 2 has a specific gravity, which is smaller than that of a liquid 10 introduced into the subject 1 by oral intake or the like, and floats on a liquid surface of the liquid 10 of a desired amount in the subject 1. In this case, the capsule medical apparatus 2 sequentially takes the in-vivo image of the subject 1 while taking a predetermined floating posture on the liquid surface of the liquid 10, and sequentially wirelessly transmits the image signal of the obtained in-vivo image to the receiving device 3.

The liquid 10 on which the capsule medical apparatus 2 floats may be any kinds of liquid as long as it is harmless to a human body, and may be, for example, at least one liquid selected from a liquid group of water, edible oil, barium solution, ionic solution, and the like. Further, timing for introducing the liquid 10 into the subject 1 may be the same as timing for introducing the capsule medical apparatus 2, or may be before or after introducing the capsule medical apparatus 2.

The receiving device 3 receives an in-vivo image group of the subject 1 taken by the capsule medical apparatus 2 in the subject 1. Specifically, the receiving device 3 has a plurality of receiving antennas 3a to 3h, which are dispersedly arranged on a body surface of the subject 1. The receiving device 3 is carried by the subject 1. The receiving antennas 3a to 3h capture a wireless signal (electric wave) from the capsule medical apparatus 2 in the subject 1. The receiving device 3 receives the electric wave from the capsule medical apparatus 2 in the subject 1 through the plurality of receiving antennas 3a to 3h, and demodulates (extracts) the image signal included in the received electric wave each time the electric wave is received. The receiving device 3 sequentially obtains image data based on the image signal, in other words, the in-vivo image of the subject 1 taken by the capsule medical apparatus 2. Further, the recording medium 5 is detachably inserted to the receiving device 3. The receiving device 3 accumulates the obtained in-vivo image group of the subject 1 in the recording medium 5.

The receiving antennas 3a to 3h may be dispersedly arranged at predetermined positions of a jacket the subject 1 wears. When the subject 1 wears the jacket, the receiving antennas 3a to 3h are dispersedly arranged on the body surface of the subject 1. The number of the receiving antennas of the receiving device 3 to be arranged on the body surface of the subject 1 is not limited to eight, as far as one or more receiving antennas are arranged.

The image display device 4 has a configuration such as that of a workstation for displaying various pieces of information such as the in-vivo image of the subject 1 taken by the capsule medical apparatus 2. Specifically, the recording medium 5 detached from the receiving device 3 is inserted to the image display device 4, and the image display device 4 takes in the in-vivo image group or the like of the subject 1 accumulated in the recording medium 5. The image display device 4 displays the in-vivo image group of the subject 1 on a display in response to operation by a user such as a doctor or a nurse. Further, the image display device 4 has a processing function for observation (examination) of the in-vivo image of the subject 1 for the diagnosis of the subject 1.

The recording medium 5 is a portable recording medium that passes data between the receiving device 3 and the image display device 4. Specifically, the recording medium 5 is attachable and detachable to/from the receiving device 3 and the image display device 4 and has such a structure that data output and data recording are possible when inserted to the receiving device 3 and the image display device 4. The recording medium 5 accumulates the in-vivo image group or the like of the subject 1 received by the receiving device 3 when inserted to the receiving device 3, and outputs the accumulated data such as the in-vivo image of the subject 1 to the image display device 4 when inserted to the image display device 4.

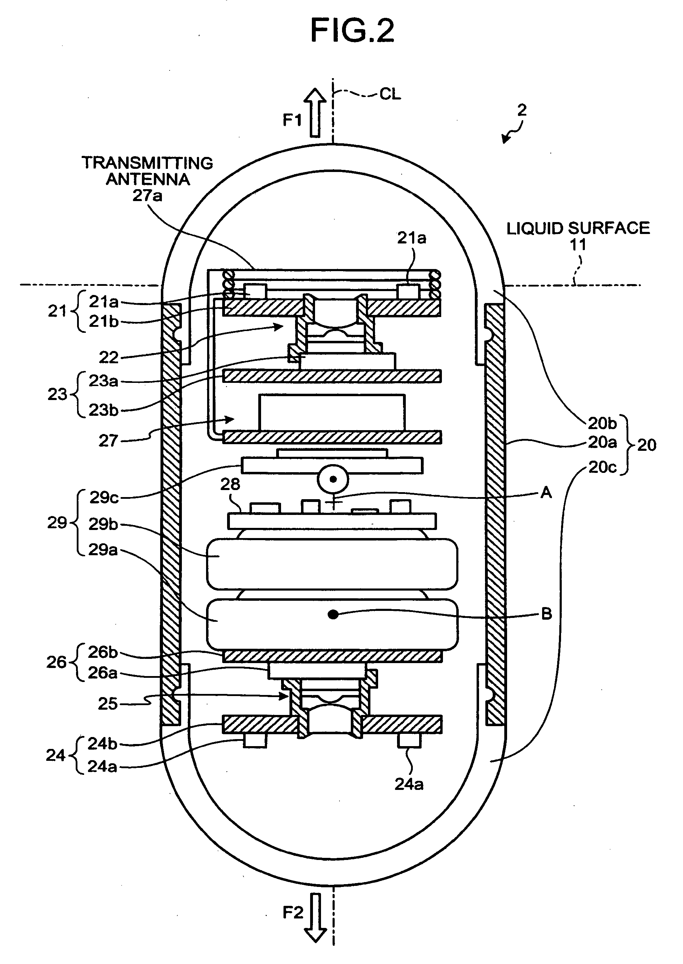

Next, a configuration of the capsule medical apparatus 2 according to the embodiment of the present invention is described in detail. FIG. 2 is a cross-sectional schematic diagram of one configuration example of the capsule medical apparatus according to the embodiment of the present invention. As illustrated in FIG. 2, the capsule medical apparatus 2 according to a first embodiment includes a capsule-type casing 20 of a size introducible into the subject 1, illuminating units 21 and 24 that illuminates an inside of the subject 1 in different directions, an optical system 22 that forms an image of an object illuminated by the illuminating unit 21, an imaging unit 23 that takes an image of an object illuminated by the illuminating unit 21, an optical system 25 that forms an image of an object illuminated by the illuminating unit 24, and an imaging unit 26 that takes an image of an object illuminated by the illuminating unit 24. In addition, the capsule medical apparatus 2 includes a transmitting unit 27 that wirelessly transmits the in-vivo images taken by the imaging units 23 and 26, a controlling unit 28 that controls the components of the capsule medical apparatus 2, and a power supply unit 29 that supplies power to the components of the capsule medical apparatus 2.

The capsule-type casing 20 is a casing of the size easily introducible (for example, easily orally taken) into the subject 1, and is formed into an outer shape with dome-shaped end portions and a cylindrical body portion as illustrated in FIG. 2. The capsule-type casing 20 is realized by blocking opening ends on both sides of a tubular casing 20a in cylindrical shape by dome-shaped casings 20b and 20c. The tubular casing 20a is a casing which is opaque to visible light. The dome-shaped casings 20b and 20c are casings which are transparent to visible light. The capsule-type casing 20 formed of the tubular casing 20a and dome-shaped casings 20b and 20c has the components (specifically, the illuminating units 21 and 24, the optical systems 22 and 25, the imaging units 23 and 26, the transmitting unit 27, the controlling unit 28 and the power supply unit 29) of the capsule medical apparatus 2 fixedly arranged therein, and liquid-tightly houses them.

The illuminating unit 21 illuminates the object of the imaging unit 23 on a side of the dome-shaped casing 20b. Specifically, the illuminating unit 21 is realized by using a plurality of light-emitting devices 21a and an illuminating substrate 21b. The plurality of light emitting devices 21a is LEDs or the like, and emits illumination light for illuminating an inside of an organ of the subject 1. The illuminating substrate 21b is a circuit substrate which has the plurality of light-emitting devices 21a mounted thereon, and is fixedly arranged in the vicinity of the dome-shaped casing 20b in the capsule-type casing 20. The illuminating unit 21 illuminates the inside of the organ of the subject 1, which is the object of the imaging unit 23, through the dome-shaped casing 20b.

The optical system 22 is realized by using a collecting lens for collecting light reflected by the object illuminated by the illuminating unit 21 and a lens frame for holding the collecting lens. The optical system 22 is fixedly arranged in a through-hole formed in the illuminating substrate 21b of the illuminating unit 21, as illustrated in FIG. 2, by being inserted thereinto. The optical system 22 collects the light reflected by the object of the imaging unit 23 on a light receiving surface of the imaging unit 23 to form the image of the object on the light receiving surface of the imaging unit 23.

The imaging unit 23 serves as an in-vivo information obtaining unit for obtaining the in-vivo image, which is one example of the in-vivo information of the subject 1. Specifically, the imaging unit 23 is realized by using a solid-state imaging device 23a such as a CCD or a CMOS image sensor and an imaging substrate 23b. The solid-state imaging device 23a is fixed to an end portion of the lens frame of the optical system 22, for imaging the image of the object formed by the optical system 22. The imaging substrate 23b is a circuit substrate which has the solid-state imaging device 23a mounted thereon, and is fixedly arranged on a side of the dome-shaped casing 20b in the capsule-type casing 20. The imaging unit 23 takes an image inside the organ located on a direction F1 side (the in-vivo image on the direction F1 side) through the dome-shaped casing 20b. Herein, the direction F1 is an imaging direction of the imaging unit 23 and is the direction on the dome-shaped casing 20b side out of directions defined by a central axis CL in a longitudinal direction of the capsule-type casing 20. More specifically, the direction F1 is the direction pointing above from a liquid surface 11 of the liquid 10 (refer to FIG. 1) on which the capsule medical apparatus 2 floats in the subject 1. The imaging unit 23 takes the in-vivo image on the direction F1 side, that is to say, the image inside the organ in air above the liquid surface 11.

The illuminating unit 24 illuminates the object of the imaging unit 26 on a side of the dome-shaped casing 20c. Specifically, the illuminating unit 24 is realized by using a plurality of light-emitting devices 24a and an illuminating substrate 24b. The plurality of light-emitting devices 24a is LEDs or the like, and emits the illumination light for illuminating an inside of an organ of the subject 1. The illuminating substrate 24b is a circuit substrate having the plurality of light-emitting devices 24a mounted thereon and is fixedly arranged in the vicinity of the dome-shaped casing 20c in the capsule-type casing 20. The illuminating unit 24 illuminates the inside of the organ of the subject 1, which is the object of the imaging unit 26, through the dome-shaped casing 20c.

The optical system 25 is realized by using a collective lens for collecting light reflected by the object illuminated by the illuminating unit 24 and a lens frame for holding the collective lens. The optical system 25 is fixedly arranged in a through-hole formed in the illuminating substrate 24b of the illuminating unit 24, as illustrated in FIG. 2, by being inserted thereinto. The optical system 25 collects the light reflected by the object of the imaging unit 26 on a light receiving surface of the imaging unit 26 to form the image of the object on the light receiving surface of the imaging unit 26.

The imaging unit 26 serves as an in-vivo information obtaining unit for obtaining the in-vivo image, which is one example of the in-vivo information of the subject 1. Specifically, the imaging unit 26 is realized by using a solid-state imaging device 26a such as a CCD or a CMOS image sensor and an imaging substrate 26b. The solid-state imaging device 26a is fixed to an end portion of the lens frame of the optical system 25, for imaging the image of the object formed by the optical system 25. The imaging substrate 26b is a circuit substrate having the solid-state imaging device 26a mounted thereon, and is fixedly arranged on a side of the dome-shaped casing 20c in the capsule-type casing 20. The imaging unit 26 takes an image inside the organ located on a direction F2 side (the in-vivo image on the direction F2 side) through the dome-shaped casing 20c. Herein, the direction F2 is an imaging direction of the imaging unit 26 and is the direction on the dome-shaped casing 20c side out of directions defined by the central axis CL of the capsule-type casing 20. More specifically, the direction F2 is the direction different from the imaging direction (direction F1) of the above-described imaging unit 23 and is the direction pointing below from the liquid surface 11 of the liquid 10 on which the capsule medical apparatus 2 floats in the subject 1. The imaging unit 26 takes the in-vivo image on the direction F2 side, that is to say, the image inside the organ in the liquid below the liquid surface 11.

The transmitting unit 27 serves as a wireless transmitting unit for transmitting the electric wave including the in-vivo information of the subject 1 to the outside. Specifically, the transmitting unit 27 is realized by using a transmitting antenna 27a, a circuit substrate, and the like. The transmitting antenna 27a is, for example, a coil antenna, and is fixedly arranged on the substrate surface of the illuminating substrate 21b and in the vicinity of an outer edge portion of the illuminating substrate 21b. In this case, the position and the direction of the transmitting antenna 27a are relatively fixed with respect to the capsule-type casing 20. Further, the transmitting antenna 27a is connected to the transmitting unit 27 body through a flexible substrate or the like.

The transmitting unit 27 wirelessly transmits the electric wave including the in-vivo images of the subject 1 taken by the imaging units 23 and 26 to the outside through the transmitting antenna 27a. More specifically, each time the transmitting unit 27 obtains the image signal of the in-vivo image on the direction F1 side taken by the imaging unit 23, the transmitting unit 27 performs predetermined modulation processing or the like on the image signal to generate a modulated signal obtained by modulating the image signal. The transmitting unit 27 outputs the modulated signal to the transmitting antenna 27a and transmits the electric wave including the modulated signal to the outside through the transmitting antenna 27a. On the other hand, each time the transmitting unit 27 obtains the image signal of the in-vivo image on the direction F2 side imaged by the imaging unit 26, the transmitting unit 27 performs predetermined modulation processing or the like on the image signal to generate a modulated signal obtained by modulating the image signal. The transmitting unit 27 outputs the modulated signal to the transmitting antenna 27a and transmits the electric wave including the modulated signal to the outside through the transmitting antenna 27a. The electric wave (electric wave including the image signal of the in-vivo image) transmitted by such a transmitting unit 27 through the transmitting antenna 27a propagates in the subject 1 and is received by the external receiving device 3 (refer to FIG. 1).

The controlling unit 28 controls operation of the components (specifically, such as the illuminating units 21 and 24, the imaging units 23 and 26, and the transmitting unit 27) of the capsule medical apparatus 2, and controls input and output of the signal among the components. Specifically, the controlling unit 28 controls operation timing of the illuminating unit 21 and the imaging unit 23 so as to take the image (in-body image on the direction F1 side) of the object illuminated by the illuminating unit 21, and controls the operation timing of the illuminating unit 24 and the imaging unit 26 so as to take the image (in-body image on the direction F2 side) of the object illuminated by the illuminating unit 24. The controlling unit 28 has various parameters relating to image processing such as white balance, and has an image processing function for sequentially generating the image signal of the in-vivo images taken by the imaging units 23 and 26. The controlling unit 28 sequentially transmits the image signal of the in-vivo image to the transmitting unit 27 and controls the transmitting unit 27 so as to sequentially transmit the electric wave including the image signal of the in-vivo image to the outside.

The power supply unit 29 supplies power to the components (specifically, such as the illuminating units 21 and 24, the imaging units 23 and 26, the transmitting unit 27, and the controlling unit 28) of the capsule medical apparatus 2. Specifically, the power supply unit 29 is realized by using two batteries 29a and 29b and a switch unit 29c. The batteries 29a and 29b are button-type batteries such as silver oxide batteries, for generating power required for operation of the components of the capsule medical apparatus 2. The switch unit 29c is, for example, a magnetic switch unit for switching between on-state and off-state by an action of a magnetic field from the outside. When the switch unit 29c is in the on-state, the power supply unit 29 supplies power of the batteries 29a and 29b to the components of the capsule medical apparatus 2, and when the switch unit 29c is in the off-state, the power supply unit 29 stops power supply.

A required number of batteries are built into the power supply unit 29, and the number is not limited to two. Further, the switch unit 29c is not limited to a magnetic switch unit and may be a switch that switches between the on-state and the off-state by predetermined light entering from the outside (such as infrared light), for example.

Herein, the capsule medical apparatus 2, which has the above-described configuration, floats on the liquid 10 in the subject 1, as illustrated in FIG. 1. Specifically, a specific gravity G of the capsule medical apparatus 2 is set to a value smaller than that of the liquid 10 through the adjustment of a total weight of the capsule medical apparatus 2 and a volume of the capsule-type casing 20. For example, when the liquid 10 is water, the specific gravity G of the capsule medical apparatus 2 is set to be smaller than the specific gravity of water (=1).

Further, a barycenter B of the capsule medical apparatus 2 is set on a predetermined position by adjusting the arrangement of the components in the capsule-type casing 20. Specifically, relatively heavy components out of the components of the above-described capsule medical apparatus 2 such as the batteries 29a and 29b are arranged on the dome-shaped casing 20c side relative to a center A of the capsule-type casing 20, and thereby the barycenter B of the capsule medical apparatus 2 is set on a position on the dome-shaped casing 20c side relative to the center A. In this case, it is desired that the barycenter B be set on a position on the central axis CL of the capsule-type casing 20.

The capsule medical apparatus 2 having such a center of gravity G and a barycenter B floats on the liquid surface 11 of the liquid 10 in the subject 1 and takes a specific floating posture on the liquid surface 11. Specifically, the capsule medical apparatus 2 takes the floating posture (that is to say, an upstanding mode) in which the dome-shaped casing 20b of the capsule-type casing 20 floats on the liquid surface 11 and in which the direction of the central axis CL of the capsule-type casing 20 and a vertical direction substantially conform to each other in the liquid 10 in a static state. In this case, the position of the liquid surface 11 around the capsule medical apparatus 2 in such a floating posture conforms to the position of the transmitting antenna 27a in the capsule-type casing 20, as illustrated in FIG. 2. Specifically, the transmitting antenna 27a is arranged on the position of the liquid surface 11 (position of a gas-liquid interface) when a part of the capsule-type casing 20 (specifically, the dome-shaped casing 20b) floats on the liquid surface 11 of the liquid 10 in the static state.

The static state of the liquid 10 used herein is a state in which the liquid 10 stays in the subject 1 (in a stomach, for example) and in which the capsule medical apparatus 2 floating on the liquid surface 11 does not fluctuate in the vertical direction. In other words, the capsule medical apparatus 2 floating on the liquid surface 11 of the liquid 10 in the static state may float in a horizontal direction with respect to the liquid surface 11 but does not fluctuate in an up and down direction (vertical direction) with respect to the liquid surface 11. On the other hand, when the liquid 10 is in a flow state, the capsule medical apparatus 2 floating on the liquid surface 11 of the liquid 10 floats in accordance with the flow of the liquid 10.

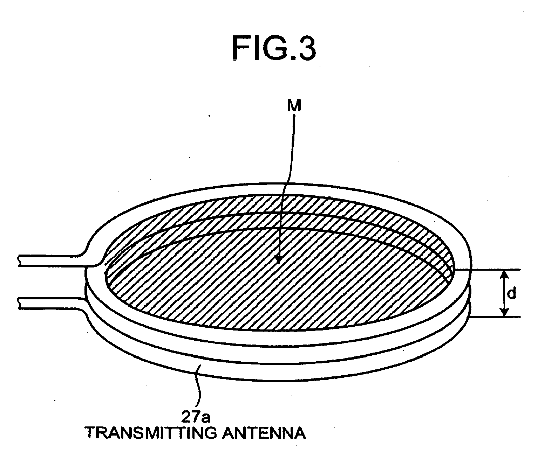

Next, the transmitting antenna 27a of the capsule medical apparatus 2 according to the embodiment of the present invention is described in detail. FIG. 3 is a schematic diagram illustrating the transmitting antenna of the capsule medical apparatus according to the embodiment of the present invention. As illustrated in FIG. 3, the transmitting antenna 27a is a coil antenna, and is realized by winding a conductive wire formed of metal such as copper or iron into a coil of predetermined number of turns (for example, three turns, or preferably two turns).

A length L (hereinafter, referred to as a conductive wire length L) of the conductive wire, which forms the transmitting antenna 27a, is set considering a wavelength of an output electric wave of the transmitting antenna 27a, that is to say, the electric wave including the above-described image signal of the subject 1. Herein, the electric wave transmitted by the transmitting unit 27 to the outside through the transmitting antenna 27a is a highfrequency signal in a frequency band of 300 to 400 MHz, for example. The wavelength of such an electric wave is shortened by a wavelength shortening effect when the electric wave propagates through a medium having a dielectric constant higher than that of the air (specifically, such as a living tissue and water). The conductive wire length L of the transmitting antenna 27a is set to approximately half the wavelength of the electric wave, in consideration of such a wavelength shortening effect. To approximate the conductive wire length L to half the wavelength of the output electric wave of the transmitting antenna 27a in this manner is effective to improve a gain of the transmitting antenna 27a.

Specifically, when the frequency of the electric wave is 315 MHz, the wavelength (=950 mm) of the electric wave is shortened to 124 mm in the living tissue (dielectric constant ε=58) in the subject 1 and is shortened to 106 mm in water (dielectric constant ε=80) in the subject 1 by the wavelength shortening effect. The conductive wire length L of the transmitting antenna 27a is set to 54 mm, for example, so as to approximate half the wavelength of the electric wave, in consideration of such a wavelength shortening effect. In this case, a ratio between the conductive wire length L and the shortened wave length (124 mm and 106 mm) of the electric wave due to the wavelength shortening effect is 0.43 and 0.51, respectively, and they approximate to 0.5. By setting the conductive wire length L in this manner, the gain of the transmitting antenna 27a can be improved.

On the other hand, a coil length d of the transmitting antenna 27a is determined depending on the conductive wire length L, the number of turns of the coil, and an outer diameter of the coil of the transmitting antenna 27a. Herein, the outer diameter of the coil of the transmitting antenna 27a is set to such a dimension that the coil can be arranged in the capsule-type casing 20. Specifically, the diameter is set to such a dimension that the coil can be arranged on the illuminating substrate 21b. As far as the coil of the transmitting antenna 27a can be arranged in the capsule-type casing 20, it is desired that the outer diameter of the coil is as larger as possible, in terms of improving the gain of the transmitting antenna 27a. Further, the number of turns of the coil of the transmitting antenna 27a is determined depending on the conductive wire length L and the outer diameter of the coil. The coil length d of the transmitting antenna 27a is determined depending on the conductive wire length L, the outer diameter of the coil, and the number of turns of the coil. When the transmitting antenna 27a of the coil length d is fixedly arranged on the illuminating substrate 21b, the transmitting antenna 27a does not block the illumination of the object by the illuminating unit 21 and the taking of the in-vivo image by the imaging unit 23.

The transmitting antenna 27a having the coil length d realized by winding the conductive wire of the above-described conductive wave length L into the coil has an opening surface M of the coil as illustrated in FIG. 3. The opening surface M of the transmitting antenna 27a is parallel to the liquid surface 11 when the capsule medical apparatus 2 floats on the liquid surface 11 of the liquid 10 in the static state. In other words, the transmitting antenna 27a is fixedly arranged in the capsule-type casing 20 such that the opening surface M and the liquid surface 11 are parallel to each other when the capsule medical apparatus takes a specific floating posture on the liquid surface 11 of the liquid 10.

The transmitting antenna 27a in the capsule-type casing 20 is arranged on the position of the liquid surface 11 in a state in which the dome-shaped casing 20b floats on the liquid surface 11 of the liquid 10 in the static state, as described above. In this case, the liquid surface 11 is located within the range from the upper end portion to the lower end portion of the transmitting antenna 27a in the capsule-type casing 20 in the floating state, that is to say, in the range of the coil length d of the transmitting antenna 27a. By arranging the transmitting antenna 27a on the position of the liquid surface 11 (that is to say, the gas-liquid interface) in this manner, the dielectric constant around the transmitting antenna 27a becomes an intermediate value of the dielectric constant of the liquid 10 and the dielectric constant of the air.

When arranging the transmitting antenna 27a on the position of the liquid surface 11 in this manner, it is desirable that the transmitting antenna 27a is in a state in which a coil portion of half or more of the coil length d sinks under the liquid surface 11 and the remaining coil portion is above the liquid surface 11.

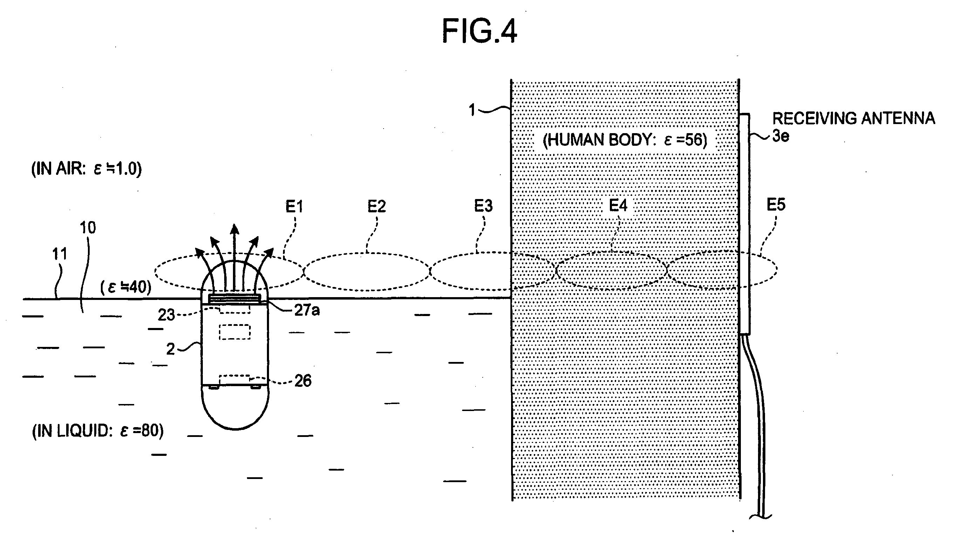

Next, an action of the capsule medical apparatus 2 according to the embodiment of the present invention on an electric wave propagation property is specifically described by illustrating a case in which the liquid 10 on which the capsule medical apparatus 2 floats in the subject 1 is water, and the electric wave from the capsule medical apparatus 2 floating on the liquid 10 is captured by the receiving antenna 3e of the external receiving device 3. FIG. 4 is a schematic diagram for illustrating a state in which the capsule medical apparatus in the floating state transmits the electric wave to the external receiving antenna through the transmitting antenna.

As illustrated in FIG. 4, the capsule medical apparatus 2 introduced into the subject 1 floats on the liquid surface 11 of the liquid 10 (such as water) in the subject 1, and maintains the specific floating posture on the liquid surface 11. Specifically, the capsule medical apparatus 2 in such a floating state takes the floating posture in which the dome-shaped casing 20b of the capsule-type casing 20 is above the liquid surface 11 and the remaining portion (on the side of the dome-shaped casing 20c) of the capsule-type casing 20 sinks below the liquid surface 11. In this case, the transmitting antenna 27a in the capsule medical apparatus 2 is arranged on the position of the liquid surface 11 of the liquid 10. Further, the opening surface M (refer to FIG. 3) of the transmitting antenna 27a is parallel to the liquid surface 11. In the floating state of the capsule medical apparatus 2, the imaging unit 23 images the in-vivo image in the air above the liquid surface 11 and the imaging unit 26 images the in-vivo image in the liquid below the liquid surface 11.

In the capsule medical apparatus 2 in the floating state, the transmitting unit 27 transmits the electric wave including the image signal of the in-vivo image taken by the imaging unit 23 or 26 through the transmitting antenna 27a, as described above. The electric wave output from the transmitting antenna 27a propagates in the subject 1 and is captured by the external receiving antenna 3e. The magnetic field emitted by the transmitting antenna 27a induces and generates an electric field, which further induces and generates a magnetic field. The electric wave from the transmitting antenna 27a reaches the external receiving antenna 3e by repeating such an electromagnetic induction.

The liquid 10 in the subject 1 is water with the dielectric constant ε=80, and above the liquid surface 11 of the liquid 10 is the air with the dielectric constant ε=1. Further, the living tissue of the subject 1 (that is to say, a human body) has the dielectric constant ε=56. In the conventional capsule medical apparatus, the electric wave output from the transmitting antenna propagates through the air with the dielectric constant ε=1.0 or water with the dielectric constant ε=80, and thereafter propagates through the living tissue with the dielectric constant ε=56 to reach the external receiving antenna 3e. In this case, since the difference in dielectric constant between the propagation media of the electric wave (water, air, and living tissue) is large, reflectance loss of the electric wave on an inner wall portion of the human body is large. Due to this, it is difficult to improve the electric wave propagation property in the conventional capsule medical apparatus.

On the other hand, the transmitting antenna 27a of the capsule medical apparatus 2 according to the embodiment of the present invention is arranged on the position of the liquid surface 11 of the liquid 10, as illustrated in FIGS. 2 and 4. Therefore, the dielectric constant around the transmitting antenna 27a is an intermediate value (about 40) of the dielectric constant of the air (ε=1.0) and the dielectric constant of the liquid 10 (ε=80). The electric wave output from the transmitting antenna 27a propagates through an area in the vicinity of the gas-liquid interface (that is to say, an area in the vicinity of the liquid surface 11), which has an intermediate dielectric constant of the air and the liquid 10, propagates through the living tissue with the dielectric constant ε=58, and reaches the external receiving antenna 3e. The difference in the dielectric constant between the propagation media (vicinity of the liquid surface 11 and the living tissue) of the electric wave from the transmitting antenna 27a is restrained to a sufficiently small difference as compared to that of the conventional capsule medical apparatus. Therefore, the reflectance loss of the electric wave on the inner wall portion of the human body may be reduced, and as a result, the electric wave propagation property at the time of the wireless transmission of the electric wave (the electric wave including the image signal of the in-vivo image) from the capsule medical apparatus 2 in the subject 1 to the receiving device 3 outside of the subject 1 may be improved.

Further, in the capsule medical apparatus 2 in the above-described floating state, the transmitting antenna 27a is arranged such that the opening surface M (refer to FIG. 3) thereof is parallel to the liquid surface 11. Therefore, the transmitting antenna 27a generates and outputs the electric wave having a polarization plane parallel to the liquid surface 11 (horizontal polarized wave). Specifically, electric field components of the electric wave output from the transmitting antenna 27a are parallel to the liquid surface 11 in the vicinity of the liquid surface 11 as illustrated as electric field components E1 to E5 in FIG. 4. This allows the capsule medical apparatus 2 to prevent the electric field components of the electric wave from a transmitting antenna 7a from going between the air and the liquid 10 between which the difference in the dielectric constant ε is large (that is to say, prevent the electric field components from passing through the gas-liquid interface). As a result, the reflectance loss of the electric wave on the gas-liquid interface (liquid surface 11) can be prevented, and thus the electric wave propagation property of the capsule medical apparatus 2 can be further improved.

As described above, the embodiment of the present invention is configured such that the specific gravity and the barycenter of the capsule medical apparatus are set such that the capsule medical apparatus floats on the liquid surface of the liquid in the subject while taking the specific floating posture, the transmitting antenna of the transmitting unit for transmitting the electric wave including the in-vivo image of the subject to the outside is fixedly arranged in the capsule-type casing, and the transmitting antenna in the capsule-type casing is arranged on the liquid surface position when the capsule medical apparatus floats on the liquid surface of the liquid in the subject. Therefore, the difference between the dielectric constant of the propagation medium of the electric wave output from the transmitting antenna and that of the human body can be reduced by making the dielectric constant around the transmitting antenna an intermediate dielectric constant of the air and the liquid, and thereby it is possible to reduce the reflectance loss of the electric wave from the transmitting antenna on the inner wall portion of the human body. As a result, the capsule medical apparatus capable of improving the electric wave propagation property when wirelessly transmitting the electric wave including the image signal of the in-vivo image from the inside of the subject to the outside can be realized.

Further, in the embodiment of the present invention, an arranging direction of the transmitting antenna in the capsule-type casing is determined such that the opening surface of the transmitting antenna in the capsule-type casing is parallel to the liquid surface when the capsule medical apparatus floats on the liquid surface of the liquid in the subject. Therefore, it becomes possible to generate and output the electric wave having the polarization plane parallel to the liquid surface (horizontal polarized wave) by the transmitting antenna, and thereby it is possible to prevent the electric field components of the electric wave from the transmitting antenna from passing through the gas-liquid interface of the air and the liquid between which the difference in dielectric constant is large. As a result, it is possible to further improve the electric wave propagation property when wirelessly transmitting the electric wave from the inside of the subject to the outside by preventing the reflectance loss of the electric wave on the gas-liquid interface.

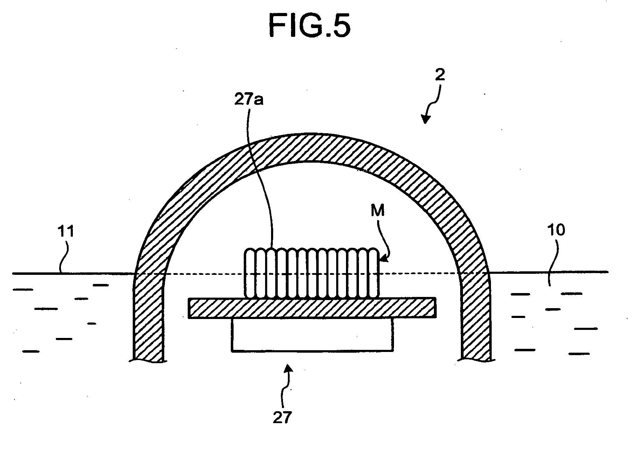

Although the transmitting antenna 27a is fixedly arranged in the capsule-type casing 20 such that the opening surface M of the transmitting antenna 27a is parallel to the liquid surface 11 in the state in which the capsule medical apparatus 2 floats on the liquid surface 11 of the liquid 10 in the subject 1 in the embodiment of the present invention, the arrangement is not limited to this, and the opening surface of the transmitting antenna 27a is not necessarily parallel to the liquid surface 11 as long as the transmitting antenna 27a is fixedly arranged in the capsule-type casing 20 such that the transmitting antenna 27a is arranged on the position of the liquid surface 11 in the capsule medical apparatus 2 in the floating state. Specifically, the transmitting antenna 27a may be fixedly arranged on the circuit substrate of the transmitting unit 27 as illustrated in FIG. 5. In this case, the opening surface M of the transmitting antenna 27a is not necessarily parallel to the liquid surface 11 and may be vertical to the liquid surface 11, for example, in the state in which the capsule medical apparatus 2 floats on the liquid surface 11 of the liquid 10. The transmitting antenna 27a in such an arrangement is arranged on the position of the liquid surface 11 when the liquid surface 11 is located within a range of an outer diameter of the opening surface M.

Further, although the transmitting antenna 27a is the coil antenna in the embodiment of the present invention, the antenna is not limited to the coil antenna. The transmitting antenna 27a of the capsule medical apparatus 2 according to the present invention may be a dipole antenna. In this case, a pole position and a pole direction of the transmitting antenna 27a in the capsule-type casing 20 are set such that the transmitting antenna 27a, which is the dipole antenna, generates and outputs the electric wave having the polarization plane parallel to the liquid surface 11.

Further, although a twin-lens capsule medical apparatus 2 provided with the two imaging units 23 and 26 having different imaging directions is illustrated in the embodiment of the present invention, the capsule medical apparatus is not limited to this. The capsule medical apparatus 2 according to the present invention may be a single-lens capsule medical apparatus provided with a single imaging unit, and may be a compound-lens capsule medical apparatus provided with three or more imaging units including two or more imaging units having different imaging directions. In this case, the imaging directions of one or more imaging units incorporated in the capsule medical apparatus 2 are not limited to the above-described directions F1 and F2 but may be desired directions. For example, the imaging direction of the imaging unit may be a direction parallel to, or vertical to, or diagonal to the central axis CL of the capsule-type casing 20. Further, one or more imaging units incorporated in the capsule medical apparatus 2 may be fixedly arranged on predetermined positions in the capsule-type casing 20. In this case, in the capsule-type casing 20, a transparent portion may be provided on a position corresponding to each imaging unit.

Although the capsule medical apparatus 2, which takes the floating posture in which the direction of the central axis CL of the capsule-type casing 20 and the vertical direction conform to each other (that is to say, the upstanding posture), is illustrated in the embodiment of the present invention, the posture is not limited to this, and the floating posture of the capsule medical apparatus 2 on the liquid surface 11 of the liquid 10 in the subject 1 may be a floating posture in which the central axis CL of the capsule-type casing 20 is parallel to the liquid surface 11 (horizontal posture) or may be a floating posture in which the central axis CL of the capsule-type casing 20 is diagonal to the liquid surface 11 (diagonal posture) as long as it is a floating posture in which the transmitting antenna 27a is arranged on the position of the liquid surface 11.

Further, although the transmitting antenna 27a is fixedly arranged in the vicinity of the dome-shaped casing 20b (specifically, in the vicinity of an outer periphery of the illuminating substrate 21b) in the embodiment of the present invention, the arrangement is not limited to this. The transmitting antenna 27a may be fixedly arranged in the vicinity of the dome-shaped casing 20c (for example, in the vicinity of the outer periphery of the illuminating substrate 24b) or may be fixedly arranged on a desired position in the tubular casing 20a (for example, in the vicinity of the inner wall portion of the tubular casing 20a) as long as it is arranged on the position of the liquid surface 11 in the capsule medical apparatus 2 in the floating state.

Although the capsule medical apparatus 2 provided with the capsule-type casing 20 having the dome-shaped end portions and the cylindrical body portion is illustrated in the embodiment of the present invention, the casing is not limited to this, and the capsule-type casing 20 of the capsule medical apparatus 2 according to the present invention may be a spherical casing or may be an elliptical casing as long as it has an outer shape easily introducible into the subject 1.

Further, although the capsule medical apparatus 2 for obtaining the in-vivo image as the in-vivo information of the subject 1 is illustrated in the embodiment of the present invention, the capsule medical apparatus is not limited to this, and it may be a capsule medical apparatus for measuring a pH value or a temperature in the subject 1 as the in-vivo information, or may be a capsule medical apparatus for detecting a state of the living tissue as the in-vivo information. Further, it may be a capsule medical apparatus having a function to spray or inject medical agent in the subject 1, or a capsule medical apparatus for collecting a substance in the living body such as living tissue.

In addition, although the position of the barycenter B of the capsule medical apparatus 2 is set by adjusting the arrangements of the batteries 29a and 29b in the capsule-type casing 20 in the embodiment of the present invention, the method is not limited to this, and the position of the barycenter B may be set by adjusting the arrangement of the components other than the batteries 29a and 29b such as the transmitting unit 27, the controlling unit 28, and the switch unit 29c, or the position of the barycenter B may be set by arrangement of a spindle member or a floating member, which are additionally arranged outside or inside of the capsule-type casing 20.

In the capsule medical apparatus according to an embodiment, the transmitting antenna is arranged at such a position that the transmitting antenna is located at the position of the liquid surface when the capsule medical apparatus is floated on the liquid surface of the liquid in the subject. Therefore, the difference between the dielectric constant around the transmitting antenna and the dielectric constant of the human body can be reduced. Thus, the reflectance loss of the electric wave at the inner wall portion inside the subject can be reduced, and as a result, electric wave propagation property can be improved in wireless transmission of the electric waves including in-vivo information from inside the subject to the outside.

## Claims

1. A capsule medical apparatus (2) adapted to float on a liquid surface of liquid (10) introduced into a subject (1), comprising:
at least one in-vivo information obtaining unit (23, 26) that obtains in-vivo information of the subject (1);
a transmitting unit (27) that wirelessly transmits an electric wave including the in-vivo information obtained by the at least one in-vivo information obtaining unit (23, 26) to outside through a transmitting antenna (27a); and
a capsule-type casing (20) having the at least one in-vivo information obtaining unit (23, 26), the transmitting antenna (27a), and the transmitting unit (27) fixedly arranged therein, the capsule-type casing (20) having a first end portion (20b) and a second end portion (20c), a central axis (CL) extending in a longitudinal direction of the capsule type casing (20), and a barycenter (B) of the capsule medical apparatus (2) being set on a position on the second end portion (20c) side relative to a center (A) of the capsule-type casing (20) and being set on a position on the central axis (CL); wherein
the transmitting antenna (27a) is fixedly arranged in the first end portion (20b) in a manner that a portion of the transmitting antenna (27a) sinks under the liquid surface of the liquid (10) and the remaining portion of the transmitting antenna (27a) is above the liquid surface so that the transmitting antenna (27a) is arranged on a liquid surface position of the liquid (10) when the first end portion (20b) floats on the liquid surface of the liquid (10).

2. The capsule medical apparatus (2) according to claim 1, wherein the transmitting antenna (27a) outputs the electric wave having a polarization plane parallel to the liquid surface of the liquid (10).

3. The capsule medical apparatus (2) according to claim 2, wherein
the transmitting antenna (27a) is a coil antenna, and
an opening surface of the coil antenna is parallel to the liquid surface of the liquid (10).

4. The capsule medical apparatus (2) according to any one of claims 1 to 3, wherein
the first end portion (20b) and the second end portion (20c) are dome-shaped,
the capsule-type casing (20) is a casing having the first end portion (20b), the second end portion (20c), and a cylindrical body portion, and
the first end portion (20b) floats on the liquid surface of the liquid (10) and the second end portion (20c) is located under the liquid surface of the liquid (10).

5. The capsule medical apparatus (2) according to any one of claims 1 to 4, wherein the at least one in-vivo information obtaining unit (23, 26) is at least one imaging unit that takes an in-vivo image being the in-vivo information of the subject (1).

6. The capsule medical apparatus according to claim 5, wherein the at least one imaging unit is a plurality of imaging units that take in-vivo images in different directions.

7. The capsule medical apparatus (2) according to claim 6, wherein
the plurality of imaging units include:
a gas side imaging unit that takes an In-vivo image above the liquid surface of the liquid (10), and
a liquid side imaging unit that takes an in-vivo image below the liquid surface of the liquid (10).

8. The capsule medical apparatus (2) according to claim 1, wherein the transmitting antenna (27a) is a coil antenna.

9. The capsule medical apparatus (2) according to claim 8, wherein the coil antenna has a coil length (d) and an opening surface (M) parallel to the liquid surface of the liquid (10), and the liquid suface of the liquid (10) is located within the range of the coil length (d) of the transmitting antenna (27a).

10. The capsule medical apparatus (2) according to claim 8, wherein the coil antenna has an opening surface (M) vertical to the liquid surface of the liquid (10), and the liquid surface of the liquid (10) is located within the range of an outer diameter of the opening surface (M).

## Patentansprüche

1. Kapselförmige medizinische Vorrichtung (2), die zum Schwimmen an einer Flüssigkeitsoberfläche einer in ein Subjekt (1) eingeführten Flüssigkeit (10) ausgebildet ist, aufweisend:
wenigstens eine in-vivo-Informationserfassungseinheit (23,26), die in-vivo-Informationen des Subjekts (1) erfasst,
eine Übertragungseinheit (27), die kabellos eine elektrische Welle zur Umgebung über eine Übertragungsantenne (27a) überträgt, wobei die elektrische Welle die von der wenigstens einen in-vivo-Informationserfassungseinheit (23,26) erfasste in-vivo-Information aufweist;
ein kapselförmiges Gehäuse (20), in dem wenigstens die in-vivo-Informationserfassungseinheit (23, 26), die Übertragungsantenne (27a) und die Übertragungseinheit (27) fest angebracht sind, wobei das kapselförmige Gehäuse (20) einen ersten Endabschnitt (20b) und einen zweiten Endabschnitt (20c), eine sich in einer Längsrichtung des kapselförmigen Gehäuses (20) erstreckende zentrale Achse (CL) und einen Schwerpunkt (B) der kapselförmigen medizinischen Vorrichtung (2) aufweist, der sich an einer Position an der Seite des zweiten Endabschnitts (20c) relativ zu einem Zentrum (A) des kapselförmigen Gehäuses (20) und an einer Position auf der zentralen Achse (CL) befindet, wobei
die Übertragungsantenne (27a) fest in dem ersten Endabschnitt (20b) derart angeordnet ist, dass ein Abschnitt der Übertragungsantenne (27a) unter die Flüssigkeitsoberfläche der Flüssigkeit (10) sinkt und der verbleibende Abschnitt der Übertragungsantenne (27a) sich oberhalb der Flüssigkeitsoberfläche befindet, so dass die Übertragungsantenne (27a) an einer Flüssigkeitsoberflächenposition der Flüssigkeit (10) angeordnet ist, wenn der erste Endabschnitt (20b) an der Flüssigkeitsoberfläche der Flüssigkeit (10) schwimmt.

2. Kapselförmige medizinische Vorrichtung (2) nach Anspruch 1, wobei die Übertragungsantenne (27a) die elektrische Welle mit einer Polarisationsebene ausgibt, die parallel zu der Flüssigkeitsoberfläche der Flüssigkeit (10) verläuft.

3. Kapselförmige medizinische Vorrichtung (2) nach Anspruch 2, wobei die Übertragungsantenne (27a) eine Spiralantenne ist, und eine Öffnungsfläche der Spiralantenne sich parallel zu der Flüssigkeitsoberfläche der Flüssigkeit (10) erstreckt.

4. Kapselförmige medizinische Vorrichtung (2), nach einem der Ansprüche 1 bis 3, wobei der erste Endabschnitt (20b) und der zweite Endabschnitt (20c) kuppelförmig ausgebildet sind,
das kapselförmige Gehäuse (20) ein Gehäuse mit dem ersten Endabschnitt (20b), dem zweiten Endabschnitt (20c) und einem zylindrischen Abschnitt ist, und
der erste Endabschnitt (20b) an der Flüssigkeitsoberfläche der Flüssigkeit (10) schwimmt und der zweite Endabschnitt (20c) sich unter der Flüssigkeitsoberfläche der Flüssigkeit (10) befindet.

5. Kapselförmige medizinische Vorrichtung (2) nach einem der Ansprüche 1 bis 4,
wobei die wenigstens eine in-vivo-Informationserfassungseinheit (23,26) wenigstens eine Bildeinheit ist, die ein in-vivo-Bild aufnimmt, das die in-vivo-Information des Subjekts (1) ist.

6. Kapselförmige medizinische Vorrichtung nach Anspruch 5, wobei die wenigstens eine Bildeinheit eine Mehrzahl von Bildeinheiten ist, die in-vivo-Bilder in verschiedenen Richtungen aufnehmen.

7. Kapselförmige medizinische Vorrichtung (2) nach Anspruch 6, wobei die Mehrzahl der Bildeinheiten umfasst:
eine gasseitige Bildeinheit, die ein in-vivo-Bild oberhalb der Flüssigkeitsoberfläche der Flüssigkeit (10) aufnimmt, und
eine flüssigkeitsseitige Bildeinheit, die ein in-vivo-Bild unterhalb der Flüssigkeitsoberfläche der Flüssigkeit (10) aufnimmt.

8. Kapselförmige medizinische Vorrichtung (2) nach Anspruch 1, wobei die Übertragungsantenne (27a) eine Spiralantenne ist.

9. Kapselförmige medizinische Vorrichtung (2) nach Anspruch 8, wobei die spiralförmige Antenne eine Spirallänge (d) und eine Öffnungsfläche (M) aufweist, die sich parallel zu der Flüssigkeitsoberfläche der Flüssigkeit (10) erstreckt, und die Flüssigkeitsoberfläche der Flüssigkeit (10) in dem Bereich der Spirallänge (d) der Übertragungsantenne (27a) angeordnet ist.

10. Kapselförmige medizinische Vorrichtung (2) nach Anspruch 8, wobei die Spiralantenne eine Öffnungsfläche (M) aufweist, die sich vertikal zur Flüssigkeitsoberfläche der Flüssigkeit (10) erstreckt, und die Flüssigkeitsoberfläche der Flüssigkeit (10) in dem Bereich des Außendurchmessers der Öffnungsfläche (M) angeordnet ist.

## Revendications

1. Appareil médical (2) de type capsule adapté pour flotter sur une surface de liquide d'un liquide (10) introduit dans un sujet (1), comprenant :
au moins une unité (23, 26) d'obtention d'informations in vivo qui obtient des informations in vivo du sujet (1) ;
une unité (27) de transmission qui transmet sans fil une onde électrique incluant les informations in vivo obtenues par la au moins une unité (23, 26) d'obtention d'informations in vivo vers l'extérieur par l'intermédiaire d'une antenne de transmission (27a) ; et
un boîtier (20) de type capsule comportant la au moins une unité (23, 26) d'obtention d'informations in vivo, l'antenne de transmission (27a), et l'unité (27) de transmission agencées de manière fixe dans celui-ci, le boîtier (20) de type capsule comportant une première partie d'extrémité (20b) et une deuxième partie d'extrémité (20c), un axe central (CL) s'étendant dans une direction longitudinale du boîtier (20) de type capsule, et un barycentre (B) de l'appareil médical (2) de type capsule étant établi sur une position du côté de la deuxième partie d'extrémité (20c) par rapport à un centre (A) du boîtier (20) de type capsule et étant établi sur une position sur l'axe central (CL) ; dans lequel
l'antenne de transmission (27a) est agencée de manière fixe dans la première partie d'extrémité (20b) d'une manière telle qu'une partie de l'antenne de transmission (27a) coule sous la surface de liquide du liquide (10) et la partie restante de l'antenne de transmission (27a) se trouve au-dessus de la surface de liquide de sorte que l'antenne de transmission (27a) est agencée à une position de surface de liquide du liquide (10) lorsque la première partie d'extrémité (20b) flotte sur la surface de liquide du liquide (10).

2. Appareil médical (2) de type capsule selon la revendication 1, dans lequel l'antenne de transmission (27a) délivre en sortie l'onde électrique ayant un plan de polarisation parallèle à la surface de liquide du liquide (10).

3. Appareil médical (2) de type capsule selon la revendication 2, dans lequel
l'antenne de transmission (27a) est une antenne à bobine, et
une surface d'ouverture de l'antenne à bobine est parallèle à la surface de liquide du liquide (10).

4. Appareil médical (2) de type capsule selon l'une quelconque des revendications 1 à 3, dans lequel
la première partie d'extrémité (20b) et la deuxième partie d'extrémité (20c) sont en forme de dôme,
le boîtier (20) de type capsule est un boîtier comportant la première partie d'extrémité (20b), la deuxième partie d'extrémité (20c), et une partie de corps cylindrique, et
la première partie d'extrémité (20b) flotte sur la surface de liquide du liquide (10) et la deuxième partie d'extrémité (20c) est placée sous la surface de liquide du liquide (10).

5. Appareil médical (2) de type capsule selon l'une quelconque des revendications 1 à 4, dans lequel la au moins une unité (23, 26) d'obtention d'informations in vivo est au moins une unité d'imagerie qui prend une image in vivo correspondant aux informations in vivo du sujet (1).

6. Appareil médical de type capsule selon la revendication 5, dans lequel la au moins une unité d'imagerie correspond à une pluralité d'unités d'imagerie qui prennent des images in vivo dans différentes directions.

7. Appareil médical (2) de type capsule selon la revendication 6, dans lequel la pluralité d'unités d'imagerie comprend :
une unité d'imagerie côté gaz qui prend une image in vivo au-dessus de la surface de liquide du liquide (10), et
une unité d'imagerie côté liquide qui prend une image in vivo au-dessous de la surface de liquide du liquide (10).

8. Appareil médical (2) de type capsule selon la revendication 1, dans lequel l'antenne de transmission (27a) est une antenne à bobine.

9. Appareil médical (2) de type capsule selon la revendication 8, dans lequel l'antenne à bobine présente une longueur de bobine (d) et une surface d'ouverture (M) parallèles à la surface de liquide du liquide (10), et la surface de liquide du liquide (10) est placée à l'intérieur de la plage de la longueur de bobine (d) de l'antenne de transmission (27a).

10. Appareil médical (2) de type capsule selon la revendication 8, dans lequel l'antenne à bobine comporte une surface d'ouverture (M) verticale par rapport à la surface de liquide du liquide (10), et la surface de liquide du liquide (10) est placée à l'intérieur de la plage d'un diamètre externe de la surface d'ouverture (M).
